# FASCICULE DE BREVET EUROPEEN

(11) **EP 1 339 337 B1**
(45) Date de publication et mention de la délivrance du brevet: **31.03.2004**
(21) Numéro de dépôt: 01999325.2
(22) Date de dépôt: 07.12.2001
(51) Int. Cl.: A61B 17/70

(54) **DISPOSITIF DE FIXATION D'UNE TIGE ET D'UNE TETE DE VIS A SYMETRIE SPHERIQUE**
VORRICHTUNG ZUR BEFESTIGUNG EINER STANGE UND EINES KUGELSYMMETRISCHEN SCHRAUBENKOPFS
DEVICE FOR FIXING A ROD AND A SPHERICAL SYMMETRY SCREW HEAD

(30) Priorité: 07.12.2000 FR 0015868; 18.05.2001 FR 0106557
(43) Date de publication de la demande: 03.09.2003
(73) Titulaire: Spine Next, La Cité Mondiale, 33000 Bordeaux (FR)
(72) Inventeur: MAZDA, Keyvan, F-75020 PARIS (FR); LE COUEDIC, Régis, F-33000 BORDEAUX (FR)
(74) Mandataire: Dronne, Guy
(86) Numéro de dépôt international: PCT/FR2001/003870
(87) Numéro de publication internationale: WO 2002/045607

(56) Documents cités:
- EP-A- 0 933 065
- WO-A-00/28907
- FR-A- 2 727 620
- FR-A- 2 784 282
- US-A- 3 253 843
- US-A- 5 344 422
- US-A- 5 437 670
- US-A- 5 584 831

## Description

La présente invention concerne un dispositif de fixation destiné à relier une tige et une tête de vis de symétrie sphérique, ladite tige étant située à proximité de ladite tête de vis.

Un domaine d'application envisagé est notamment celui de la chirurgie de la colonne vertébrale et plus particulièrement de la stabilisation du rachis pour pallier certains inconvénients liés aux accidents traumatiques ou aux patrologies dégénératives.

Des dispositifs de stabilisation comportant des vis pédiculaires et une tige de liaison apte à relier la tête desdites vis entre elles sont bien connus. Les vis pédiculaires de ces dispositifs présentent généralement une tête de réception en forme de "U" apte à recevoir la tige et la paroi interne de la tête est taraudée dans l'axe de ladite vis de façon à permettre le vissage d'une pièce de blocage contre ladite tige. Ainsi, la tige est rendue solidaire de ladite vis par serrage direct de la pièce de blocage qui forme étau avec le fond de la tête de réception.

Cependant, la force de liaison entre une tête de réception et la tige qui la traverse est déterminée par la force de serrage qui a été transmise à la pièce de blocage. Cette force de blocage est directement liée à la force physique de l'opérateur, ce qui est susceptible de varier selon les individus.

Par ailleurs, dans ces dispositifs, la vis et la tige de liaison sont reliées ensemble et présentent des directions relatives déterminées par la forme de la tête de réception.

Afin de pallier ce dernier inconvénient, il a été imaginé une tête de réception en "U" présentant une surface externe de symétrie sphérique dans laquelle une tige de liaison filetée vient s'engager de façon à visser deux écrous de chaque côté de la tête et à former étau pour maintenir en position fixe la vis pédiculaire et la tige de liaison. Ainsi, la tête de vis étant de symétrie sphérique, la tige est susceptible d'être orientée dans différentes positions par rapport à la vis, ces différentes positions étant limitées au plan de symétrie contenant le corps de la vis et séparant les deux branches de la tête de vis en forme de "U".

En outre, les deux écrous formant étau présentent également l'inconvénient de la force de serrage puisque les écrous sont directement vissés contre la tête de réception. D'autres dispositifs de fixation sont décrit dans les documents US-A-5,344,422 et US-A-5,584,831 (Le préambule de la revendication 1 est basé sur ce document.

La présente invention a pour but de remédier à ces inconvénients en proposant un dispositif de fixation dont la force de serrage est accrue.

Ce but est atteint grâce au fait que le dispositif comprend une première pièce longitudinale présentant une première surface opposée à une seconde surface, la première extrémité de ladite première surface étant apte à s'appliquer contre au moins une portion d'une première partie hémisphérique de ladite tête de vis et la partie médiane de ladite seconde surface étant apte à prendre appui contre au moins une portion d'une première partie de ladite tige ; une secondes pièce longitudinale, aptes à coopérer avec ladite première pièce longitudinale, dont la première extrémité est apte à s'appliquer, au regard d'au moins une portion de ladite première extrémité de ladite première surface de ladite première pièce, contre au moins une portion de la deuxième partie hémisphérique de ladite tête de vis opposée à la première et dont la partie médiane est apte à prendre appui contre au moins une portion d'une deuxième partie de ladite tige opposée à la première ; et comprend des premiers moyens de solidarisation et de réglage aptes à maintenir les secondes extrémités desdites première et seconde pièces longitudinales en regard et à les rapprocher l'une de l'autre de façon à faire pivoter ladite seconde pièce longitudinale autour de ladite tige par rapport à ladite première pièce longitudinale pour coincer simultanément, ladite tête de vis entre ladite première extrémité de ladite première surface et ladite première extrémité de ladite seconde pièce longitudinale, et ladite tige entre ladite partie médiane de ladite seconde surface de ladite première pièce longitudinale et la partie médiane de ladite seconde pièce longitudinale, par quoi ledit dispositif relie ladite tige et ladite tête de vis et les maintient en position fixe l'un par rapport à l'autre.

Ainsi, une caractéristique du dispositif de fixation réside dans le mode d'immobilisation et de serrage de la tête de réception et de la tige par le pivotement de deux pièces longitudinales, l'une par rapport à l'autre autour de la tige de façon que les premières extrémités desdites pièces longitudinales forme une première pince apte à coincer la tête de vis de symétrie sphérique et que les parties médianes forment une seconde pince apte à coincer simultanément la tige. Le pivotement des deux pièces longitudinales autour de la tige est réalisé grâce auxpremiers moyens de solidarisation et de réglage qui permettent de rapprocher à force, les secondes extrémités desdites pièces longitudinales l'une vers l'autre. De la sorte, par un effet de levier, la force exercée pour rapprocher l'une vers l'autre les secondes extrémités permet aux premières extrémités des pièces longitudinales de former étau et de coincer simultanément la tête de la vis et la tige avec une force bien supérieure à celle qui est obtenue par le serrage direct d'une pièce de blocage contre la tige.

En outre, la tête de vis de symétrie sphérique ne présentant pas d'échancrure dans laquelle serait introduite la tige puisqu'elle est maintenue à proximité de cette dernière, elle forme une véritable rotule et les possibilités d'orientation de la tige par rapport à la vis pédiculaire ne sont plus limités à un plan.

Selon un mode préférentiel, la première extrémité de ladite première surface forme une calotte sphérique dont le fond présente un perçage débouchant dans l'extrémité de la seconde surface de ladite pièce longitudinale, ladite calotte sphérique étant apte à s'appliquer sur ladite tête de vis. Ainsi, la première extrémité est parfaitement calée contre la tête de vis puisque l'extrémité de la surface épouse parfaitement la surface de la tête. Par ailleurs, le perçage qui est ménagé dans la pièce au fond de la calotte sphérique permet d'accéder au sommet de la tête de vis qui présente un logement hexagonal destiné au vissage de la vis pédiculaire. Ainsi, l'ajustement du serrage de la vis dans la vertèbre est rendu possible même si le dispositif de fixation est déjà monté sur la tête de vis.

Avantageusement, la partie médiane de ladite seconde surface présente un premier logement dans lequel ladite portion d'une première partie de ladite tige est apte à prendre appui. De la sorte, la tige est bloquée en translation selon la direction de la première pièce longitudinale et permet un meilleur blocage de la tige par rapport à la tête.

Selon une disposition particulièrement avantageuse, la seconde pièce longitudinale présente une portion située au regard de ladite seconde surface de ladite première pièce longitudinale et une portion recourbée formant ladite première extrémité de ladite seconde pièce longitudinale. De cette façon, la seconde pièce longitudinale, en appui sur la tige est apte à pivoter autour de cette dernière dans les limites du contact avec la première pièce longitudinale. Lorsque, la première pièce longitudinale est en appui contre une première partie hémisphérique de la tête de vis, la portion recourbée de ladite seconde pièce longitudinale s'applique contre la deuxième partie hémisphérique de la tête de vis de façon à la coincer.

De façon avantageuse, la première extrémité de ladite seconde pièce longitudinale présente une portion de calotte sphérique tronquée apte à s'appliquer contre ladite portion de la deuxième partie hémisphérique de ladite tête de vis. Ainsi, la forme de l'extrémité de ladite seconde pièce longitudinale coïncide parfaitement avec la surface de la tête de vis et permet une meilleure solidarisation.

Préférentiellement, ladite portion située au regard de ladite seconde surface de ladite première pièce longitudinale présente un perçage débouchant au regard dudit perçage débouchant dans l'extrémité de la seconde surface de ladite pièce longitudinale, par quoi la dite tête de vis est accessible lorsque ledit dispositif est monté.

Selon un mode particulier de réalisation, ladite partie médiane de ladite seconde pièce longitudinale présente un deuxième logement situé au regard dudit premier logement de ladite première pièce longitudinale, ledit second logement étant apte à recevoir ladite tige. Ainsi, ladite tige est également bloquée en translation parallèlement à ladite seconde pièce longitudinale. De la sorte, la tige est apte à être coincée entre les deux pièces longitudinales.

De façon avantageuse, lesditspremiers moyens de solidarisation et de réglage comprennent une vis apte à traverser lesdites secondes extrémités desdites première et seconde pièces longitudinales dont la tête s'applique contre ladite extrémité de ladite seconde pièce longitudinale et dont le corps est vissé dans un taraudage de la seconde extrémité de ladite première pièce longitudinale. Ainsi, le serrage de la vis tend à rapprocher et à maintenir ensemble les secondes extrémités des pièces longitudinales dont les parties médianes prennent appui pour former pivot, respectivement, contre au moins une portion d'une première partie de ladite tige et contre au moins une portion d'une deuxième partie de ladite tige opposée à la première de façon que les premières extrémités desdites pièces longitudinales enserrent la tête de vis pour bloquer l'ensemble.

Selon un premier mode particulier de mise en oeuvre, l'invention est appliquée à un ensemble de stabilisation du rachis comprenant, deux vis pédiculaires à tête sphérique aptes à être vissées chacune, dans un pédicule d'une vertèbre ; deux dispositifs de fixation conforme à l'invention montés sur lesdites têtes de vis, lesdites pièces longitudinales étant sensiblement parallèles entre elles et à l'axe du rachis ; une tige transversale apte à relier lesdits deux dispositifs entre eux, ladite tige étant sensiblement perpendiculaire à l'axe du rachis ; et, un système de connexion apte à relier ladite tige transversale à une tige longitudinale sensiblement parallèle à l'axe du rachis.

Ainsi, l'ensemble de stabilisation selon ce premier mode particulier de réalisation permet de maintenir entre elles les vertèbres d'une portion de rachis lorsque les organes anatomiques aptes à remplir cette fonction font défaut. Pour ce faire, les dispositifs de fixation conformes à l'invention montés sur chacune des deux vis pédiculaires fixées dans une vertèbre sont reliés ensemble par une tige transversale formant échelon sur la vertèbre et les échelons sont reliés ensemble par des tiges longitudinales au moyen d'un système de connexion apte à solidariser deux tiges sensiblement perpendiculaires.

Selon un deuxième mode particulier de mise en oeuvre, l'invention est appliquée à un ensemble de stabilisation du rachis comprenant, deux vis pédiculaires à tête sphérique aptes à être vissées dans les pédicules de deux vertèbres superposées ; deux dispositifs de fixation selon l'invention, montés sur lesdites têtes de vis, lesdites pièces longitudinales étant sensiblement parallèles entre elles et perpendiculaires à l'axe du rachis ; et, une tige longitudinale apte à relier lesdits dispositifs entre eux, ladite tige longitudinale étant sensiblement parallèle à l'axe du rachis.

De la sorte, les dispositifs conformes à l'invention sont montés sur deux vis pédiculaires fixées dans deux vertèbres successives, d'un même côté du rachis, et ils sont reliés par une tige de façon à maintenir dans une position fixe les deux pédicules en vis-à-vis de deux vertèbres successives. Afin de renforcer symétriquement la colonne vertébrale un ensemble analogue est monté sur l'autre côté du rachis.

Selon un troisième mode particulier de mise en oeuvre, l'invention est appliquée à un ensemble de stabilisation du rachis comprenant, au moins deux vis pédiculaires à tête sphérique aptes à être vissées chacune, dans un pédicule d'une vertèbre, deux dispositifs, conformes à l'invention, montés sur lesdites têtes de vis, lesdites secondes extrémités desdites pièces longitudinales étant dirigées l'une vers l'autre, et, une pièce de liaison transversale apte à relier lesdits deux dispositifs entre eux, ladite pièce de liaison étant solidaire d'au moins une seconde extrémité desdites pièces desdits deux dispositifs.

Tout comme dans le mode particulier de mise en oeuvre précédent, le dispositif de fixation conforme à l'invention permet de relier des vis pédiculaires superposées avec des tiges disposées parallèlement à l'axe du rachis. Cependant, les dispositifs sont, dans ce troisième mode particulier, reliés entre eux, transversalement d'un côté à l'autre du rachis, par une pièce de liaison transversale.

Selon un quatrième mode particulier de mise en oeuvre de l'invention, le dispositif de fixation comprend en outre, des seconds moyens de solidarisation réglables, solidaires de ladite seconde pièce longitudinale et situés entre sa première extrémité et sa partie médiane, lesdits seconds moyens de solidarisation étant susceptibles de prendre appui contre la première extrémité de ladite seconde surface de ladite première pièce longitudinale de façon à rapprocher l'une de l'autre, ladite première extrémité de la seconde pièce longitudinale et ladite portion de ladite première extrémité de ladite première surface de ladite première pièce, pour emprisonner ladite tête de vis, par quoi lesdites pièces longitudinales sont susceptibles de pivoter l'une par rapport à l'autre autour de ladite tête de vis de symétrie sphérique, entre une position où lesdites secondes extrémités desdites pièces longitudinales sont écartées l'une de l'autre et une position où lesdites secondes extrémités desdites pièces longitudinales sont rapprochées l'une de l'autre.

Ainsi, cette caractéristique, outre qu'elle permet un meilleur blocage de la tête de vis de symétrie sphérique, permet également de solidariser de façon non démontable les premières extrémités des pièces longitudinales sans avoir à relier, avec lesdits premiers moyens de solidarisation et de réglage, lesdites secondes extrémités desdites première et seconde pièces longitudinales. De la sorte, la tige est susceptible d'être ajustée par rapport aux deux pièces longitudinales, ces dernières restant solidaires de la tête de vis de symétrie sphérique, avant d'être bloquée par le rapprochement desdites secondes extrémités desdites pièces longitudinales au moyen des premiers moyens de solidarisation.

Avantageusement, lesdits second moyens de solidarisation sont constitués d'un élément vissable susceptible de coopérer avec un taraudage ménagé dans la paroi dudit premier perçage, ledit élément vissable étant susceptible de prendre appui contre la première extrémité de ladite seconde surface de ladite première pièce longitudinale. De la sorte, l'entraînement en rotation dudit élément vissable, l'entraîne en translation dans la direction de la première extrémité de ladite seconde surface de ladite première pièce ou dans la direction contraire.

De façon préférentielle, ladite seconde extrémité de ladite seconde pièce longitudinale présente un évidement formant U débouchant dans la direction opposée à ladite première extrémité, ladite vis traversant de façon incliné ladite seconde extrémité de ladite première pièce longitudinale de façon que ladite tête de vis soit éloignée de ladite première extrémité lorsqu'elle est dans un état dévissée et qu'elle soit rapprochée de ladite première extrémité dans un état vissée par quoi ladite vis est susceptible d'être engagé dans ledit évidement formant U, ladite tête de vis en appui contre le pourtour dudit évidement.

Ainsi, selon cette caractéristique, la seconde pièce longitudinale est susceptible d'être entraînée en pivotement autour de la tête de vis en écartant lesdites secondes extrémités l'une de l'autre, lorsque lesdits seconds moyens de solidarisation réglables sont mis en oeuvre, alors que la vis est maintenue en position dévissée dans ladite première pièce longitudinale, la tête de vis éloignée de la première extrémité. De la sorte, la vis peut être engagée avant le montage du dispositif et serrée ensuite pour bloquer ladite tige, comme on l'expliquera plus en détails dans la suite de la description.

De façon particulièrement avantageuse, ladite première pièce longitudinale présente en outre un second perçage, situé entre ladite partie médiane et ladite seconde extrémité de ladite première pièce longitudinale, débouchant respectivement dans lesdites première et seconde surface et susceptible de recevoir une vis dont la tête s'applique contre ladite seconde surface pour maintenir ladite première pièce longitudinale en position fixe par rapport à une paroi, ladite première surface contre ladite paroi. Cette caractéristique particulière, conformément au quatrième mode de réalisation de l'invention permet de relier ledit dispositif de fixation à la paroi latérale des corps vertébraux.

Avantageusement, le dispositif conforme à ce quatrième mode de mise en oeuvre de l'invention est appliqué à un ensemble de stabilisation du rachis comprenant : au moins deux vis postérieures à tête sphérique susceptibles d'être vissées, dans deux vertèbres superposées, chacune dans la paroi latérale postérieure du corps vertébrale de la vertèbre ; et, au moins deux dispositifs de fixation selon la revendication, lesdites premières pièces longitudinales étant susceptibles d'être maintenues en position fixe sensiblement parallèlement entre elles et aux plans moyens desdites vertèbres, contre la paroi latérale des vertèbres par des vis antérieures traversant ledit second perçage, les premières extrémités des premières surfaces étant respectivement en appui contre lesdites têtes sphériques et les deux dispositifs de fixation étant maintenus en position fixe l'un par rapport à l'autre par une seule tige coincée respectivement par les parties médianes desdites pièces longitudinales. Ainsi, les deux vertèbres sont maintenues l'une par rapport à l'autre, ledit ensemble permettant, notamment, de suppléer partiellement de disque intervertébral.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels:
- la Figure 1 est une vue schématique en coupe verticale du dispositif de fixation selon l'invention reliant la tige en coupe perpendiculaire et la tête de symétrie sphérique ;
- la Figure 2, est une vue éclatée de l'invention montrant les différents moyens qui la constitue ;
- la Figure 3 est une vue schématique en perspective montrant un ensemble de stabilisation comprenant un dispositif de fixation conforme à l'invention ;
- la Figure 4 est une vue schématique montrant un ensemble de stabilisation comprenant un dispositif de fixation conforme à l'invention, selon un mode particulier de mise en oeuvre.
- la Figure 5 est une vue schématique en coupe verticale du dispositif de fixation selon un autre mode particulier de mise en oeuvre de l'invention, en position déverrouillée ;
- la Figure 6 est une vue schématique en coupe verticale du dispositif de fixation selon l'autre mode particulier de mise en oeuvre de l'invention, en position verrouillée ;
- la Figure 7 est une vue éclatée du dispositif de fixation selon l'autre mode particulier de mise en oeuvre de l'invention; et,
- la Figure 8 est une vue en coupe du dispositif de fixation conforme à l'invention et de la vertèbre dans laquelle il est inséré.

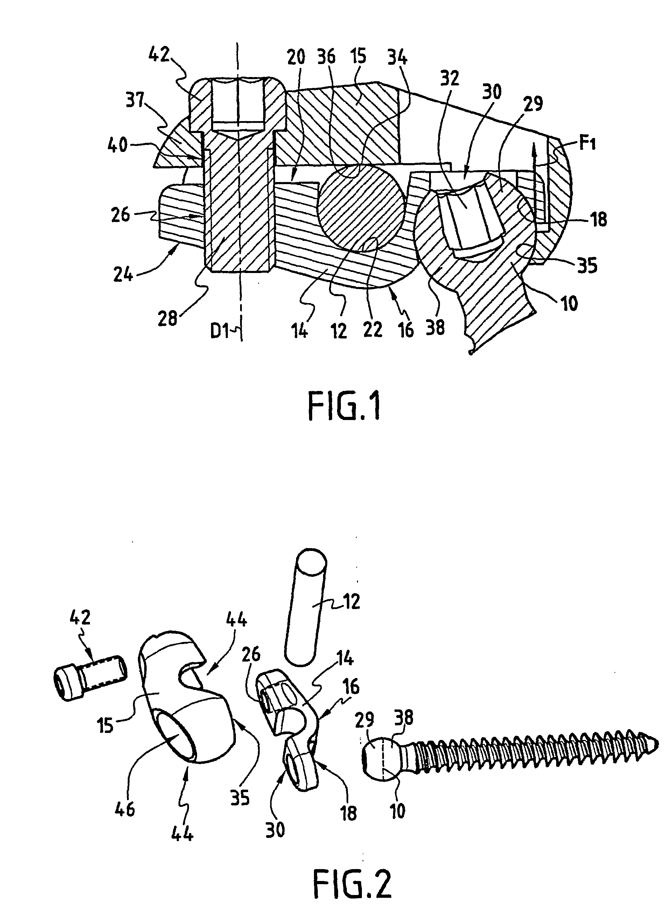

On se référera tout d'abord à la Figure 1 pour décrire le dispositif de fixation conforme à l'invention et son mode de fonctionnement.

Le dispositif de fixation conforme à l'invention permet de maintenir ensemble dans une position relative déterminée, une tête de vis 10 de symétrie sphérique et une tige 12, situées à proximité l'une de l'autre. Le dispositif comprend une première pièce longitudinale 14 et une seconde pièce longitudinale 15 disposée en regard de la première. La première pièce longitudinale 14 présente une première surface 16, ou surface inférieure, contre une première extrémité 18 de laquelle vient s'appuyer la tête de vis 10. Elle présente également une seconde surface 20, ou surface supérieure, contre la partie médiane 22 de laquelle la tige 12 prend appui. A sa seconde extrémité 24, la première pièce longitudinale 14 comporte un taraudage 26 dans lequel une vis 28 est apte à être vissée.

La première extrémité 18 de la première surface 16 forme une calotte sphérique dans laquelle au moins une portion d'une première partie hémisphérique 29 de la tête de vis vient s'appliquer. Cette particularité permet de former une articulation entre le dispositif de fixation et la tête de vis. Comme on l'expliquera plus en détails dans la suite de la description cette articulation présente un avantage pour le montage de ce dispositif.

Par ailleurs, le fond de la première extrémité 18 formant calotte présente un perçage 30 traversant la première pièce longitudinale 14 de part en part pour former un anneau sphérique de façon à dégager l'extrémité de la tête de vis 10 qui présente un logement hexagonal axial 32 destiné au vissage de la vis pédiculaire dans la vertèbre.

La partie médiane 22 de la première pièce longitudinale 14 forme un logement sensiblement hémicylindrique dans lequel la tige 12 est en appui. Ce logement permet le guidage de la tige 12 dans le dispositif de fixation durant le montage et une meilleure solidarisation de la tige et du dispositif. '

Ledit logement présente des dimensions et en particulier une profondeur, par rapport à la seconde surface 20, inférieure au diamètre de la tige 12 de manière à ce qu'une portion 34 de tige 12 fasse saillie de la seconde surface 20. Ainsi, la seconde pièce longitudinale 15 située au regard de la seconde surface 20 est apte à s'appliquer contre la portion 34 de tige 12 qui forme un pivot pour la seconde pièce longitudinale 15.

Cette dernière, appliquée au regard de la seconde surface 20 de la première pièce longitudinale 14 présente une première extrémité recourbée 35, une partie médiane 36 et une seconde extrémité 37. La première extrémité recourbée 35 vient s'appliquer sur au moins une portion d'une deuxième partie hémisphérique 38 de la tête de vis 10, opposée à la première partie 29, en regard d'au moins une portion de la première extrémité 18 de la première surface 16. De la sorte, la tête de vis 10 de symétrie sphérique, forme rotule pour le dispositif de fixation dont les pièces longitudinales présentent au moins quatre points d'appui dont trois sont non alignés et appliqués contre la première partie hémisphérique 29 et dont le quatrième est appliqué contre la deuxième partie hémisphérique 38 opposée à la première.

Cependant, bien que le dispositif de fixation et la tête de vis 10 sont aptes à former une articulation, et que cette caractéristique présente un avantage, le dispositif doit pouvoir être bloqué sur la tête 10.

Ainsi, la partie médiane 36 de la seconde pièce longitudinale 15 est en appui sur la portion 34 de tige 12 et la seconde extrémité 37 est située au regard de la seconde extrémité 24 de la première pièce longitudinale 14. En outre, la seconde pièce longitudinale 15, conformément à la Figure 1, ne présente pas d'autre point de contact avec la première pièce longitudinale 14, que la portion 34 de tige 12, susceptible de bloquer le pivotement de cette seconde pièce longitudinale 15 autour de la tige 12. Par ailleurs, la seconde pièce longitudinale 15 présente un perçage 40 pratiqué dans sa seconde extrémité 37 apte à venir au regard du taraudage 26 ménagé dans la seconde extrémité 24 de la première pièce longitudinale 14. Ainsi, la tête 42 de la vis 28 qui est insérée dans le perçage 40 et vissée dans le taraudage 26 de façon que la tête 42 s'applique contre l'extrémité 37 de la seconde pièce longitudinale 15.

De la sorte, le vissage de la vis 28 dans le taraudage 26 tend à rapprocher les secondes extrémités 24 et 37 des pièces longitudinales, 14 et 15. Lorsque la tête 10 de la vis pédiculaire est logée entre l'extrémité 18 de la première surface 16 et l'extrémité recourbée 35 de, que la tige 12 est insérée entre le logement de la partie médiane 22 de la première pièce longitudinale 14 et la partie médiane 36 de la seconde pièce longitudinale 15, et que les deux extrémités 24 et 37 des deux pièces longitudinales 14 et 15 sont maintenues à une distance fixe, l'une de l'autre par la vis 42 sans que cette dernière exerce une force apte à les rapprocher, le dispositif assure la liaison entre la tête de vis 10 et la tige 12.

Tant que la vis 42 n'est pas serrée dans le taraudage 26 et qu'elle ne comprime pas les deux pièces longitudinales, le dispositif est susceptible de tourner autour de la tête de vis 10 puisque l'extrémité 18 de la première surface 16 et l'extrémité 35 de la seconde pièce longitudinale 14 n'enserrent pas la tête de vis 10.

Le but principale que poursuit la présente invention est de maintenir une tige dans une position fixe par rapport à une tête de vis de symétrie sphérique, avec une force de maintien supérieure à la force de maintien obtenue avec les dispositifs de l'art antérieur, tout en exerçant une même force de serrage sur un élément vissé.

Ainsi, lorsque la vis 42 est entraînée en rotation, l'extrémité 37 et 24 des deux pièces longitudinales 14 et 15 sont rapprochées l'une de l'autre selon une direction D1, et si la première pièce longitudinale 14 et la tige 12 sont considérés comme un repère fixe, la seconde pièce longitudinale va pivoter autour de la tige 12 et l'extrémité de cette pièce 35 va comprimer la tête 10, selon F1, contre la première extrémité 18 de la première pièce longitudinale 14. Simultanément, la tige 12 est comprimée entre les parties médiane 22 et 36 des pièces longitudinales 14 et 15.

Lorsque la vis 42 est serrée avec une première intensité, l'articulation formée par le dispositif solidaire de la tige et la tête de la vis 10 est partiellement bloquée et il est possible, manuellement de donner une autre orientation à la tige 12 par rapport à la tête 10. Cette particularité permet un ajustement préalable de la tige et présente un avantage lors du montage du dispositif de fixation comme on l'expliquera dans la suite de la description.

Lorsque la position définitive est déterminée, un serrage de la vis 42 avec une deuxième intensité, supérieure à la première, est susceptible de bloquer l'articulation avec suffisamment de force pour que la tige 12 et la tête de vis 10 soient en position fixe malgré les contraintes fonctionnelles auxquelles l'articulation est soumise.

On comprend que l'effet de levier, procurer par le dispositif conforme à l'invention, permet un serrage plus important de la tige 12 et de la tête de vis 10 ensemble que ne le permet un serrage direct d'une pièce contre l'autre.

On se référera maintenant à la Figure 2 pour décrire plus particulièrement la seconde pièce longitudinale 15 dont la vue en coupe de la Figure 1 n'illustre qu'une partie.

On retrouve sur la Figure 2, la vis pédiculaire surmontée d'une tête sphérique 10, au-dessus de laquelle la première extrémité 18 de la première pièce longitudinale 14 est apte à s'appliquer. La seconde pièce longitudinale 15 forme un capotage dans lequel la première pièce longitudinale 14 est apte à être encastrée et dont les évidements latéraux 44 permettent le passage de la tige 12. Ainsi, les deux pièces longitudinales 14 et 15 sont solidaires en translation selon un axe parallèle à la tige 12.

La première extrémité 35 de la seconde pièce longitudinale 15 présente une portion de calotte sphérique tronquée susceptible de s'appliquer contre une portion de la deuxième partie hémisphérique 38 de la tête de vis.

En outre, avantageusement on prévoit un usinage déterminé de La première extrémité 35 de la seconde pièce longitudinale 15 de façon que, lorsque le dispositif est monté sur la tige 12 et que la vis 42 est partiellement vissée dans le taraudage 26, le dispositif puisse s'emboîter à force sur la tête de vis 10. Cette disposition permet de monter partiellement le dispositif et de le fixer ensuite sur les vis solidaires des vertèbres.

Par ailleurs, on retrouve sur la Figure 2 le perçage 30 qui traverse la première pièce longitudinale 14 pour accéder au logement hexagonale de vissage de la vis pédiculaire. Afin d'accéder à ce logement lorsque le dispositif de fixation complet est monté sur la tête de vis 10, la seconde pièce longitudinale 15 présente également un perçage 46 situé en regard du perçage 30.

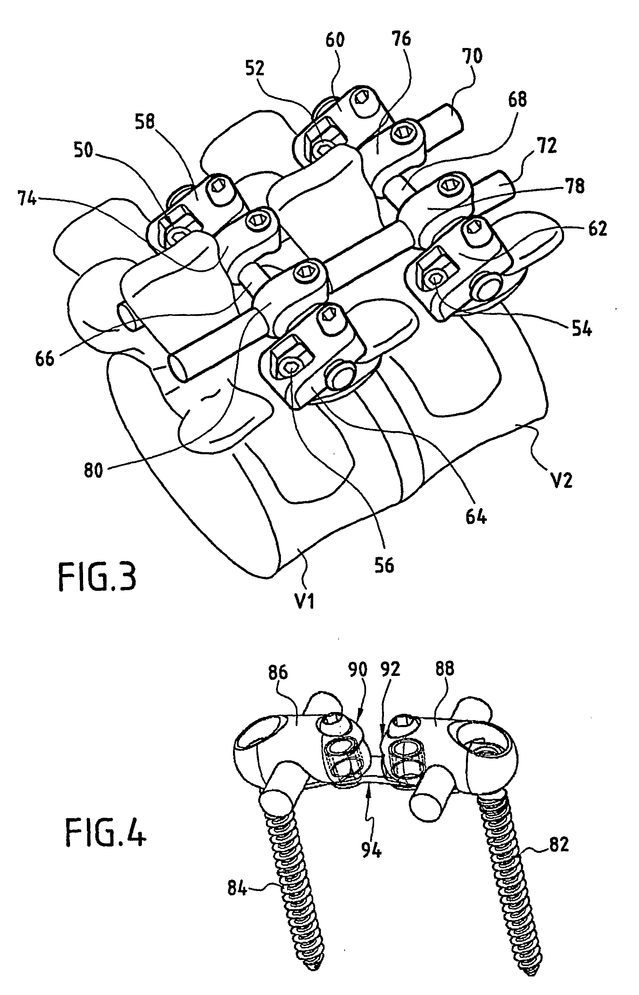

On se référera maintenant à la Figure 3 pour décrire un ensemble de stabilisation du rachis comprenant un dispositif de fixation conforme à la présente invention.

La Figure 3 illustre une portion de rachis présentant deux vertèbres, V1 et V2 dans chacune desquelles deux vis pédiculaires dont les têtes 50, 52, 53, 54 apparaissent. Sur chacune des têtes de vis 50, 52, 53, 54, un dispositif de fixation 58, 60, 62, 64 conforme à l'invention est emboîté et deux tiges transversales 66 et 68 relient respectivement les dispositifs 58 et 64 et les dispositifs 60 et 62, perpendiculairement à l'axe du rachis.

Par ailleurs, les tiges transversales 66 et 68 sont reliées entre elles par deux tiges longitudinales 70 et 72 disposées de chaque côté des apophyses épineuses. Les tiges longitudinales 70, 72, sont solidarisées aux tiges transversales 68, 66 par des dispositifs de connexion 74, 76, 78, 80. Chaque dispositif de connexion comprend un organe de connexion apte à maintenir les tiges transversales 66, 68, contre les tiges longitudinales et, des moyens de blocage réglables, aptes à s'appliquer contre les tiges longitudinales 70, 72 pour former levier et comprimer les tiges transversales de façon à bloquer ensemble les tiges et les dispositifs de connexion.

Cet ensemble permet une stabilisation optimale de la portion de rachis qui en est équipée.

Par ailleurs, lors du montage de l'ensemble de stabilisation, les vis pédiculaires sont insérées dans les vertèbres puis les dispositifs sont emboîtés deux à deux avec une tige transversale dans chacune des têtes de vis des vertèbres de la portion du rachis à stabiliser, sans serrage excessif des dispositifs de façon à faire jouer l'articulation durant le montage. Ce n'est qu'après le montage des tiges longitudinales au moyens des dispositifs de connexion que le serrage des dispositifs est effectué de façon à ce que l'ensemble s'adapte parfaitement à la morphologie du rachis ou à la forme que l'on souhaite lui donner.

Selon un mode particulier de mise en oeuvre de l'invention, l'ensemble de stabilisation du rachis comprend : deux vis pédiculaires à tête sphérique aptes à être vissées dans les pédicules de deux vertèbres superposées et deux dispositifs de fixation conformes à l'invention montés sur lesdites têtes de vis, lesdites pièces longitudinales étant sensiblement parallèles entre elles et perpendiculaires à l'axe du rachis. Par ailleurs, une tige longitudinale relie lesdits dispositifs entre eux, ladite tige longitudinale étant sensiblement parallèle à l'axe du rachis.

On se référera maintenant à la Figure 4 pour décrire un autre ensemble de stabilisation du rachis comprenant au moins deux vis pédiculaires 82, 84 à tête sphérique susceptibles d'être vissées chacune, dans un pédicule d'une vertèbre et deux dispositifs 86, 88, conformes à la présente invention, montés sur lesdites têtes de vis 82, 84, les secondes extrémités, 90 et 92 des dispositifs 86 et 88 étant dirigées l'une vers l'autre de façon que les tiges soient parallèles à l'axe du rachis. En outre, les secondes extrémités 90 et 92 sont reliées entre elles par une pièce de liaison transversale 94 perpendiculaire à l'axe du rachis.

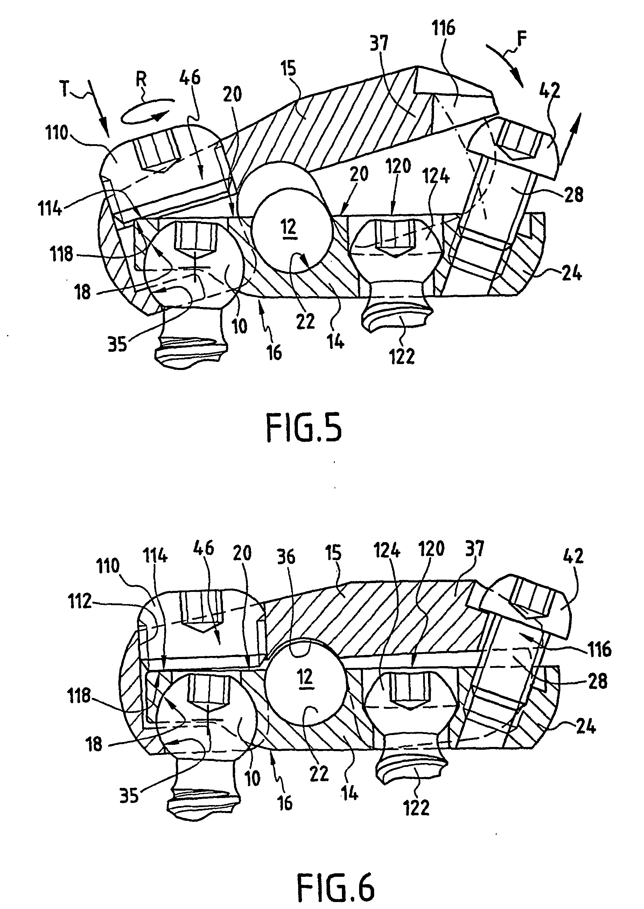

Selon un autre mode de mise en oeuvre de l'invention, son objet se fixe sur la paroi latérale des corps vertébraux et permet de maintenir les vertèbres, les unes par rapport aux autres. On se référera aux Figures 5, 6, 7 et 8 pour décrire l'invention selon ce mode de mise en oeuvre.

La Figure 5 illustre un dispositif de fixation conforme à l'invention monté sur une tête de vis 10 de symétrie sphérique, et pour lequel la première pièce longitudinale 14 est maintenu par une seconde vis 122. Comme on l'expliquera plus en détails, ces vis permettent d'ancrer le dispositif de fixation dans la paroi des corps vertébraux.

On retrouve sur la Figure 5 la tête de vis 10 de symétrie sphérique sur laquelle est en appui la première extrémité 18 de ladite première surface 16. En outre la première pièce longitudinale 14 présente un second perçage 120, situé entre la partie médiane 22 et la seconde extrémité 24 de la première pièce longitudinale 14. Une vis 122 est insérée dans le perçage 120 et la tête de vis 124 est appliquée contre la seconde surface 20 dans un logement spécialement ménagé dans la pièce. Ainsi, non seulement, la première pièce longitudinale 14 est en appui sur la tête de vis 10, mais elle est maintenue en position fixe contre une paroi, non représentée, au moyen de la vis 122 qui est vissée dans cette paroi, tout comme la vis dont la tête 10 est sphérique.

Par ailleurs, le dispositif de fixation comprend des seconds moyens de solidarisation réglables constitués d'un élément vissable 110, et d'un taraudage 112 ménagé dans la paroi interne du premier perçage 46. L'élément vissable 110 étant susceptible d'être vissé dans le taraudage 112 et d'être entraîné en translation selon l'axe du premier perçage 46. L'élément vissable 110 est donc solidaire de la seconde pièce longitudinale 15 et son extrémité inférieur 118 est susceptible de prendre appui contre la première extrémité 114 de la seconde surface 20. Ainsi, l'actionnement en rotation de l'élément vissable 110 selon R tend à rapprocher l'une de l'autre la première extrémité 35 et la portion de la première extrémité 18 et d'emprisonner la tête de vis 10. De la sorte, moyennant un serrage modéré de l'élément vissable 110 la seconde pièce longitudinale 15 est susceptible d'être écartée de la première pièce longitudinale 14, comme illustré sur la Figure 5, leurs premières extrémités 18, 35 étant maintenues autour de la tête de vis 10 et leurs secondes extrémités 24, 37 respectives étant éloignées l'une de l'autre. La tige 12 peut, ainsi, coulisser librement contre la partie médiane 22 de la seconde surface 20.

En outre, la vis 28 est susceptible d'être engagée de manière inclinée dans la seconde extrémité 24 de la première pièce longitudinale 14 et, ainsi, de permettre de maintenir la tête de vis 42 éloignée de la première extrémité 35 lorsque la vis 28 est partiellement engagée, comme l'illustre la Figure 5. De la sorte, la seconde extrémité 37 de la seconde pièce longitudinale, qui présente un évidement 116 formant U, est susceptible d'être rapprochée de la seconde extrémité 24 de la première pièce longitudinale 14, en pivotant autour de la tête 10 de symétrie sphérique. L'évidement 116 formant U débouche dans la direction opposée à la première extrémité 35, ainsi, lorsque la seconde extrémité 24 est rabattue selon la flèche F contre la première pièce longitudinale, 14 la vis 28 occupe l'évidement 116. Après vissage complet de la vis 28, sa tête 42 vient en appui contre le pourtour de l'évidement 116 et bloque les secondes extrémités 24, 37 des pièces longitudinales ensemble, comme illustré sur la Figure 6. En outre, les parties médianes 22, 36, respectivement des première et seconde pièces longitudinales enserrent et bloquent la tige 12.

Le serrage complet du dispositif de fixation est réalisé en serrant la vis 110 dans le perçage 46. La tige 12 est alors maintenue en position fixe par rapport à la paroi dans laquelle la vis à tête sphérique 10 et la vis 122 sont insérée.

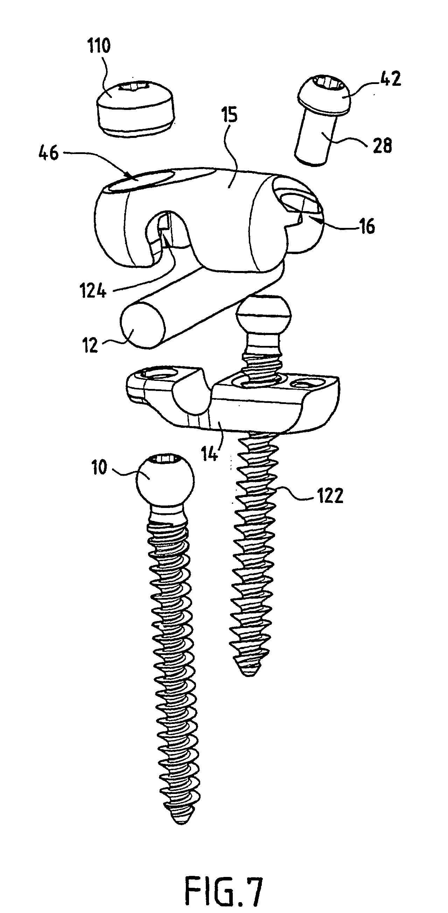

La Figure 7 illustre le dispositif de fixation conforme à l'invention en vue éclatée. On retrouve, sur cette Figure 7, la vis 122 et la vis à tête sphérique 10, permettant de fixer la première pièce longitudinale 14 sur la paroi. La vis 28 dont la tête 42 est susceptible de s'appliquer contre le pourtour de l'évidement 116, est généralement pré-engagée dans la première pièce longitudinale 14 avant le montage sur la paroi. De la même façon, l'élément vissable 110 est également prémonté dans le perçage 46 de la seconde pièce longitudinale 15 avant le montage du dispositif.

Selon un mode particulièrement avantageux de mise en oeuvre de l'invention, la partie médiane 36 de ladite seconde pièce longitudinale 15 présente des moyens de coincement 124, permettant de coincer partiellement la tige 12 de façon à la rendre partiellement solidaire de la seconde pièce longitudinale 15.

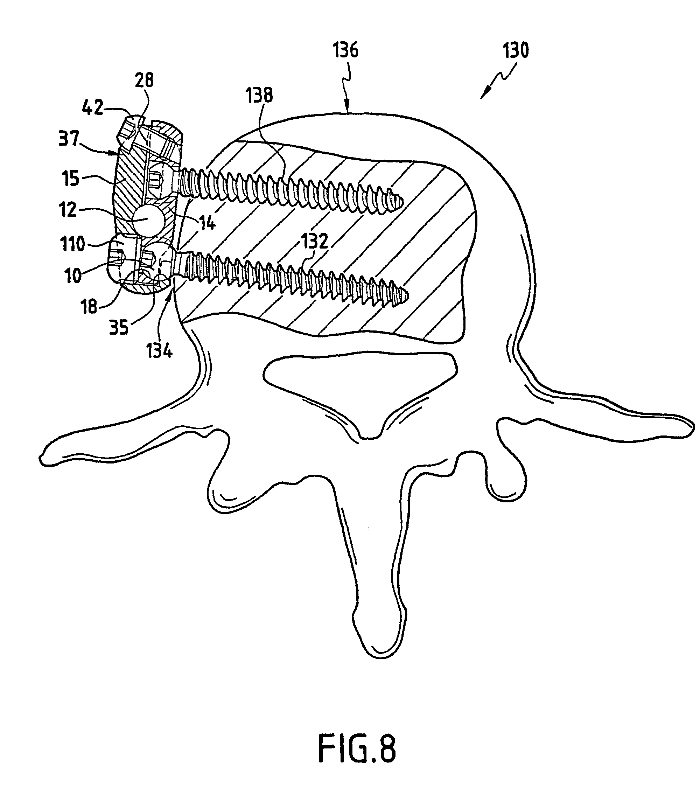

Ainsi, le dispositif de fixation conforme à l'invention qui est susceptible d'être ancré dans la paroi latérale des corps vertébraux comme l'illustre la Figure 8, est installée plus aisément sur le rachis du patient. Des dispositifs conforment à l'invention sont généralement destinés à maintenir en position fixe au moins deux vertèbres l'une par rapport à l'autre, en ancrant deux dispositifs conformes à l'invention, un dans chacune des parois latérales des vertèbres et de façon superposée de manière à pouvoir les relier par une tige.

La Figure 8 illustre le mode de fixation d'un dispositif dans une vertèbre 130.

Lors du montage du dispositif, on fixe tout d'abord les vis postérieures 132 à tête sphérique 10 dans la paroi latérale postérieure 134 du corps vertébral 136 de la vertèbre 130. Ensuite, on fixe les premières pièces longitudinales, prééquipée d'une vis 28 de solidarisation et de réglage, contre la paroi latérale des vertèbres au moyen de vis antérieure 138, la première extrémité 18 de la première surface en appui contre la tête sphérique 10. Puis, on vient engager la première extrémité 35 de la seconde pièce longitudinale 14 contre la tête sphérique 10, la seconde pièce longitudinale 14 étant prééquipée d'un élément vissable 110 et de la tige 12, coincée dans les moyens de coincement. Ainsi, on vient serrer partiellement l'élément vissable 110 pour solidariser les premières extrémités 18, 35 sur la tête sphérique 10 et on fait pivoter la seconde pièce longitudinale de façon à engager la seconde extrémité 37 sous la tête de vis 42 afin de pouvoir serrer la vis 28 et bloquer la tige 12, le serrage complet étant achevé par le serrage de l'élément vissable 110.

Bien évidement, ces mêmes opérations sont réalisées sensiblement simultanément pour les dispositifs des autres vertèbres. Lorsque les premiers moyens de solidarisation et de réglage, et les seconds moyens de solidarisation et de réglage de tous les dispositifs reliés par une même tige, sont serrés, les vertèbres dans lesquelles ils sont ancrés sont toutes maintenues en position fixe les unes par rapport aux autres.

## Revendications

1. Dispositif de fixation destiné à relier une tige et une tête de vis de symétrie sphérique, ladite tige étant située à proximité de ladite tête de vis, **caractérisé en ce qu'**il comprend :
- une première pièce longitudinale (14) présentant une première surface (16) opposée à une seconde surface (20), la première extrémité (18) de ladite première surface (16) étant apte à s'appliquer contre au moins une portion d'une première partie hémisphérique (29) de ladite tête de vis (10) et la partie médiane (22) de ladite seconde surface (20) étant apte à prendre appui contre au moins une portion d'une première partie de ladite tige (12) ;
- une secondes pièce longitudinale (15), aptes à coopérer avec ladite première pièce longitudinale (14), dont la première extrémité (35) est apte à s'appliquer, au regard d'au moins une portion de ladite première extrémité (18) de ladite première surface (16) de ladite première pièce (14), contre au moins une portion de la deuxième partie hémisphérique (38) de ladite tête de vis (10) opposée à la première (29) et dont la partie médiane (36) est apte à prendre appui contre au moins une portion (34) d'une deuxième partie de ladite tige (12) opposée à la première ;
et **en ce qu'**il comprend des premiers moyens de solidarisation et de réglage (26, 28) aptes à maintenir les secondes extrémités (24, 37) desdites première (14) et seconde (15) pièces longitudinales en regard et à les rapprocher l'une de l'autre de façon à faire pivoter ladite seconde pièce longitudinale (15) autour de ladite tige (12) par rapport à ladite première pièce longitudinale (14) pour coincer simultanément, ladite tête de vis (10) entre ladite première extrémité (18) de ladite première surface (16) et ladite première extrémité (35) de ladite seconde pièce longitudinale (15), et ladite tige (12) entre ladite partie médiane (22) de ladite seconde surface (20) de ladite première pièce longitudinale (14) et la partie médiane (36) de ladite seconde pièce longitudinale (15).

2. Dispositif de fixation selon la revendication 1, **caractérisé en ce qu'**il comprend en outre, des seconds moyens de solidarisation réglables (110, 112), solidaires de ladite seconde pièce longitudinale (15) et situés entre sa première extrémité (35) et sa partie médiane, lesdits seconds moyens de solidarisation (110, 112) étant susceptibles de prendre appui contre la première extrémité de ladite seconde surface (20) de ladite première pièce longitudinale (14) de façon à rapprocher l'une de l'autre, ladite première extrémité (35) de la seconde pièce longitudinale (15) et ladite portion de ladite première extrémité (18) de ladite première surface (16) de ladite première pièce (14), pour emprisonner ladite tête de vis (10), par quoi lesdites pièces longitudinales (14, 15) sont susceptibles de pivoter l'une par rapport à l'autre autour de ladite tête de vis (10) de symétrie sphérique, entre une position où lesdites secondes extrémités (24, 37) desdites pièces longitudinales (14, 15) sont écartées l'une de l'autre et une position où lesdites secondés extrémités (24, 37) desdites pièces longitudinales (14, 15) sont rapprochées l'une de l'autre.

3. Dispositif de fixation selon la revendication 1 ou 2, **caractérisé en ce que** ladite seconde pièce longitudinale (15) présente une première portion située au regard de ladite seconde surface (20) de ladite première pièce longitudinale (14) et une seconde portion recourbée formant ladite première extrémité (35) de ladite seconde pièce longitudinale (15).

4. Dispositif de fixation selon la revendication 3, **caractérisé en ce que** ladite première portion présente un premier perçage la traversant de part en part et débouchant au regard de ladite première extrémité de ladite seconde surface de ladite première pièce longitudinale(14).

5. Dispositif de fixation selon la revendication 2 et 4,
**caractérisé en ce que** lesdits second moyens de solidarisation sont constitués d'un élément vissable (110) susceptible de coopérer avec un taraudage (112) ménagé dans la paroi dudit premier perçage (46), ledit élément vissable (110) étant susceptible de prendre appui contre la première extrémité (114) de ladite seconde surface (20) de ladite première pièce longitudinale (14).

6. Dispositif de fixation selon l'une quelconque des revendications 1 à 5, **caractérisé en ce que** lesdits premiers moyens de solidarisation et de réglage comprennent une vis (28) apte à traverser lesdites secondes extrémités (24, 37) desdites première (14) et seconde (15) pièces longitudinales dont la tête (42) s'applique contre ladite extrémité (37) de ladite seconde pièce longitudinale (15) et dont le corps est vissé dans un taraudage (26) de la seconde extrémité (24) de ladite première pièce longitudinale (14).

7. Dispositif de fixation selon la revendication 6, **caractérisé en ce que** ladite seconde extrémité (37) de ladite seconde pièce longitudinale (15) présente un évidement (116) formant U débouchant dans la direction opposée à ladite première extrémité (35), ladite vis (28) traversant de façon incliné ladite seconde extrémité (24) de ladite première pièce longitudinale (14) de façon que ladite tête de vis (42) soit éloignée de ladite première extrémité (35) lorsqu'elle est dans un état dévissée et qu'elle soit rapprochée de ladite première extrémité (35) dans un état vissée par quoi ladite vis (28) est susceptible d'être engagé dans ledit évidement (116) formant U, ladite tête (42) de vis en appui contre le pourtour dudit évidement (116).

8. Dispositif de fixation selon l'une quelconque des revendications 1 à 7, **caractérisé en ce que** ladite première extrémité (18) de ladite première surface (16) forme une calotte sphérique dont le fond présente un perçage (30) débouchant dans l'extrémité de la seconde surface (20) de ladite pièce longitudinale (14), ladite calotte sphérique étant apte à s'appliquer sur ladite tête de vis (10).

9. Dispositif de fixation selon l'une quelconque des revendications 1 à 8, **caractérisé en ce que** ladite première extrémité (35) de ladite seconde pièce longitudinale (15) présente une portion de calotte sphérique tronquée apte à s'appliquer contre ladite portion de la deuxième partie hémisphérique (38) de ladite tête de vis (10).

10. Dispositif de fixation selon l'une quelconque des revendications 1 à 9, **caractérisé en ce que** ladite partie médiane (22) de ladite seconde surface (20) présente un premier logement dans lequel ladite portion d'une première partie de ladite tige est apte à prendre appui.

11. Dispositif de fixation selon l'une quelconque des revendications 1 à 10, **caractérisé en ce que** ladite partie médiane (36) de ladite seconde pièce longitudinale (15) présente un deuxième logement (44) situé au regard dudit premier logement, ledit second logement (44) étant apte à recevoir ladite tige (12).

12. Dispositif de fixation selon l'une quelconque des revendications 1 ou 11, **caractérisé en ce que** ladite première pièce longitudinale (14) présente en outre un second perçage (120), situé entre ladite partie médiane (22) et ladite seconde extrémité (24) de ladite première pièce longitudinale (14), débouchant respectivement dans lesdites première (16) et seconde (20) surfaces et susceptible de recevoir une vis (122) dont la tête (124) s'applique contre ladite seconde surface pour maintenir ladite première pièce longitudinale (14) en position fixe par rapport à une paroi, ladite première surface (16) contre ladite paroi.

13. Ensemble de stabilisation du rachis **caractérisé en ce qu'**il comprend :
- deux vis pédiculaires à tête sphérique (50, 56) aptes à être vissées chacune, dans un pédicule d'une vertèbre (V1) ;
- deux dispositifs (58, 64), selon l'une quelconque des revendications 1 à 11, montés sur lesdites têtes de vis (50, 56), lesdites pièces longitudinales étant sensiblement parallèles entre elles et à l'axe du rachis ;
- une tige transversale (66) apte à relier lesdits deux dispositifs (58, 64) entre eux, ladite tige (66) étant sensiblement perpendiculaire à l'axe du rachis ; et,
- un système de connexion (74, 80) apte à relier ladite tige transversale (66) à une tige longitudinale (70, 72) sensiblement parallèle à l'axe du rachis.

14. Ensemble de stabilisation du rachis **caractérisé en ce qu'**il comprend :
- deux vis pédiculaires à tête sphérique aptes à être vissées dans les pédicules de deux vertèbres superposées ;
- deux dispositifs de fixation selon l'une quelconque des revendications 1 à 11, montés sur lesdites têtes de vis, lesdites pièces longitudinales étant sensiblement parallèles entre elles et perpendiculaires à l'axe du rachis ; et,
- une tige longitudinale apte à relier lesdits dispositifs entre eux, ladite tige longitudinale étant sensiblement parallèle à l'axe du rachis.

15. Ensemble de stabilisation du rachis **caractérisé en ce qu'**il comprend :
- au moins deux vis pédiculaires (84, 82) à tête sphérique aptes à être vissées chacune, dans un pédicule d'une vertèbre ;
- deux dispositifs (86, 88), selon l'une quelconque des revendications 1 à 11, montés sur lesdites têtes de vis (84, 82), lesdites secondes extrémités (90, 92) desdites pièces longitudinales étant dirigées l'une vers l'autre; et,
- une pièce de liaison transversale (94) apte à relier lesdits deux dispositifs (86, 88) entre eux, ladite pièce de liaison étant solidaire d'au moins une seconde extrémité (90, 92) desdites pièces desdits deux dispositifs (86, 88).

16. Ensemble de stabilisation du rachis **caractérisé en ce qu'**il comprend :
- au moins deux vis postérieures à tête sphérique susceptibles d'être vissées, dans deux vertèbres superposées, chacune dans la paroi latérale postérieure du corps vertébrale de la vertèbre ; et,
- au moins deux dispositifs de fixation selon la revendication 12, lesdites premières pièces longitudinales étant susceptibles d'être maintenues en position fixe sensiblement parallèlement entre elles et aux plans moyens desdites vertèbres, contre la paroi latérale des vertèbres par des vis antérieures traversant ledit second perçage, les premières extrémités des premières surfaces étant respectivement en appui contre lesdites têtes sphériques et les deux dispositifs de fixation étant maintenus en position fixe l'un par rapport à l'autre par une seule tige coincée respectivement par les parties médianes desdites pièces longitudinales.

## Claims

1. A fixing device for connecting a rod and a nearby spherically symmetrical screwhead, which fixing device is **characterised in that** it includes:
- a first longitudinal member (14) having a first surface (16) and an opposite second surface (20), a first end (18) of said first surface (16) being adapted to be pressed against at least a portion of a first hemispherical part (29) of said screwhead (10) and a middle part (22) of said second surface (20) being adapted to bear against at least a portion of a first part of said rod (12);
- a second longitudinal member (15) adapted to co-operate with said first longitudinal member (14), whose first end (35) is adapted to be pressed, opposite at least a portion of said first end (18) of said first surface (16) of said first part (14), against at least a portion of a second hemispherical part (38) of said screwhead (10) opposite the first hemispherical part (29) and whose middle part (36) is adapted to bear against at least a portion (34) of a second part of said rod (12) opposite the first;
and **in that** it includes first fastening and adjustment means (26, 28) adapted to hold the second ends (24, 37) of said first and second longitudinal members (14, 15) facing each other and to move them toward each other so as to cause said second longitudinal member (15) to pivot around said rod (12) relative to said first longitudinal member (14) to grip said screwhead (10) between said first end (18) of said first surface (16) and simultaneously to grip said first end (35) of said second longitudinal member (15) and said rod (12) between said middle part (22) of said second surface (20) of said first longitudinal member (14) and the middle part (36) of said second longitudinal member (15).

2. A fixing device according to claim 1, **characterised in that** it further includes second adjustable fastening means (110, 112) fastened to said second longitudinal member (15) and situated between its first end (35) and its middle part, said second fastening means (110, 112) being adapted to bear against the first end of said second surface (20) of said first longitudinal member (14) so as to move said first end (35) of said second longitudinal member (15) and said portion of said first end (18) of said first surface (16) of said first member (14) toward each other to trap said screwhead (10), whereby said longitudinal members (14, 15) are adapted to pivot relative to each other about said spherically symmetrical screwhead (10) between a position in which said second ends (24, 37) of said longitudinal members (14, 15) are spaced apart from each other and a position in which said second ends (24, 37) of said longitudinal members (14, 15) are close to each other.

3. A fixing device according to claim 1 or claim 2, **characterised in that** said second longitudinal member (15) has a first portion situated opposite said second surface (20) of said first longitudinal member (14) and a curved second portion forming said first end (35) of said second longitudinal member (15).

4. A fixing device according to claim 3, **characterised in that** said first portion has a first bore passing completely through it and opening opposite said first end of said second surface of said first longitudinal member (14).

5. A fixing device according to claims 2 and 4, **characterised in that** said second fastening means consist of a screwthreaded member (110) adapted to co-operate with an internal screwthread (112) formed in the wall of said first bore (46), said screwthreaded member (110) being adapted to bear against the first end (114) of said second surface (20) of said first longitudinal member (14).

6. A fixing device according to any one of claims 1 to 5, **characterised in that** said first fastening and adjustment means include a screw (28) adapted to pass through said second ends (24, 37) of said first and second longitudinal members (14, 15) and whose head (42) is pressed against said end (37) of said second longitudinal member (15) and whose body is screwed into an internal screwthread (26) in the second end (24) of said first longitudinal member (14).

7. A fixing device according to claim 6, **characterised in that** said second end (37) of said second longitudinal member (15) has a U-shaped recess (116) opening in the direction opposite said first end (35), said screw (28) passing in an inclined manner through said second end (24) of said first longitudinal member (14) so that said screwhead (42) is spaced apart from said first end (35) when it is unscrewed and close to said first end (35) when it is screwed in, whereby said screw (28) is adapted to be engaged in said U-shaped recess (116) with said screwhead (42) bearing against the perimeter of said recess (116).

8. A fixing device according to any one of claims 1 to 7, **characterised in that** said first end (18) of said first surface (16) forms a spherical socket whose bottom includes a bore (30) opening onto the end of the second surface (20) of said longitudinal member (14), said spherical socket being adapted to be pressed onto said screwhead (10).

9. A fixing device according to any one of claims 1 to 8, **characterised in that** said first end (35) of said second longitudinal member (15) has a truncated spherical socket portion adapted to be pressed against said portion of the second hemispherical part (38) of said screwhead (10).

10. A fixing device according to any one of claims 1 to 9, **characterised in that** said middle part (22) of said second surface (20) includes a first housing in which said portion of a first part of said rod is adapted to bear.

11. A fixing device according to any one of claims 1 to 10, **characterised in that** said middle part (36) of said second longitudinal member (15) includes a second housing (44) adapted to receive said rod (12) and situated opposite said first housing.

12. A fixing device according to either claim 1 or claim 11, **characterised in that** said first longitudinal member (14) further includes a second bore (120) situated between said middle part (22) and said second end (24) of said first longitudinal member (14), respectively opening onto said first and second surfaces (16, 20) and adapted to receive a screw (122) whose head (124) is pressed against said second surface to hold said first longitudinal member (14) in a fixed position relative to a wall with said first surface (16) against said wall.

13. A spine stabilizing system **characterised in that** it includes:
- two spherical head pedicular screws (50, 56) each adapted to be screwed into a pedicle of a vertebra (V1);
- two devices (58, 64) according to any one of claims 1 to 11 mounted on said screwheads (50, 56) with said longitudinal members substantially parallel to each other and to the axis of the spine;
- a transverse rod (66) substantial perpendicular to the axis of the spine adapted to join said two devices (58, 64) together; and
- a connecting system (74, 80) adapted to connect said transverse rod (66) to a longitudinal rod (70, 72) substantially parallel to the axis of the spine.

14. A spine stabilizing system **characterised in that** it includes:
- two spherical head pedicular screws adapted to be screwed into the pedicles of two superposed vertebrae;
- two fixing devices according to any one of claims 1 to 11 mounted on said two screwheads with said longitudinal members substantially parallel to each other and perpendicular to the axis of the spine; and
- a longitudinal rod substantially parallel to the axis of the spine adapted to connect said devices together.

15. A spine stabilizing system **characterised in that** it includes:
- at least two spherical head pedicular screws (84, 82) each adapted to be screwed into a pedicle of a vertebra;
- two devices (86, 88) according to any one of claims 1 to 11 mounted on said screwheads (84, 82) with said second ends (90, 92) of said longitudinal members directed towards each other; and
- a transverse connecting part (94) fastened to at least a second end (90, 92) of said parts of said two devices (86, 88) and adapted to connect said two devices (86, 88) together.

16. A spine stabilizing system **characterised in that** it includes:
- at least two spherical head posterior screws each adapted to be screwed into the posterior lateral wall of the vertebral body of a respective one of two superposed vertebrae; and
- at least two fixing devices according to claim 12, and in which system said first longitudinal members are adapted to be held in a fixed position relative to each other, substantially parallel to each other and to the median planes of said vertebrae, and against the lateral wall of the vertebrae by anterior screws passing through said second bore, the first ends of the first surfaces respectively bearing against said spherical heads, and the two fixing devices being held in a fixed position relative to each other by a single rod gripped by the respective median parts of said longitudinal members.

## Patentansprüche

1. Befestigungsvorrichtung zum Verbinden einer Stange und eines kugelsymmetrischen Schraubenkopfes, wobei die Stange in der Nähe des Schraubenkopfes gelegen ist, **dadurch gekennzeichnet, dass** sie folgendes enthält:
- ein erstes Längsteil (14), das eine erste Fläche (16) aufweist, die einer zweiten Fläche (20) gegenüberliegt, wobei das erste Ende (18) der ersten Fläche (16) ausgebildet ist, um sich an wenigstens einen Abschnitt eines ersten halbkugelförmigen Teils (29) des Schraubenkopfes (10) anzulegen, und wobei der Mittelteil (22) der zweiten Fläche (20) ausgebildet ist, um sich an wenigstens einem Abschnitt eines ersten Teils der Stange (12) abzustützen;
- ein zweites Längsteil (15), welches ausgebildet ist, um mit dem ersten Längsteil (14) zusammenzuwirken, dessen erstes Ende (35) ausgebildet ist, um sich gegenüber wenigstens einem Abschnitt des ersten Endes (18) der ersten Fläche (16) des ersten Teils (14) an wenigstens einen Abschnitt des zweiten halbkugelförmigen Teils (38) des Schraubenkopfes (10), das dem ersten (29) gegenüberliegt, anzulegen, und dessen Mittelteil (36) ausgebildet ist, um sich an wenigstens einem Abschnitt (34) eines zweiten Teils der Stange (12), der dem ersten gegenüberliegt, abzustützen;
- und dass sie erste Mittel zum festen Verbinden und Einstellen (26, 28) aufweist, welche ausgebildet sind, um die zweiten Enden (24, 37) des ersten (14) und zweiten (15) Längsteils gegenüber zu halten und um sie derart einander zu nähern, dass das zweite Längsteil (15) um die Stange (12) gegenüber dem ersten Längsteil (14) verschwenkt wird, um gleichzeitig den Schraubenkopf (10) zwischen dem ersten Ende (18) der ersten Fläche (16) und dem ersten Ende (35) des zweiten Längsteils (15) sowie die Stange (12) zwischen dem Mittelteil (22) der zweiten Fläche (20) des ersten Längsteils (14) und dem Mittelteil (36) des zweiten Längsteils (15) einzuklemmen.

2. Befestigungsvorrichtung nach Anspruch 1, **dadurch gekennzeichnet, dass** sie außerdem zweite einstellbare Mittel zum festen Verbinden (110, 112) aufweist, die mit dem zweiten Längsteil (15) fest verbunden und zwischen dessen ersten Ende (35) und dessen Mittelteil gelegen sind, wobei die zweiten Mittel zum festen Verbinden (110, 112) geeignet sind, sich an dem ersten Ende der zweiten Fläche (20) des ersten Längsteils (14) derart abzustützen, dass das erste Ende (35) des zweiten Längsteils (15) und der Abschnitt des ersten Endes (18) der ersten Fläche (16) des ersten Teils (14) einander genähert werden, um den Schraubenkopf (10) zu umschließen, wodurch die Längsteile (14, 15) um den kugelsymmetrischen Schraubenkopf (10) zwischen einer Position, in der die zweiten Enden (24, 37) der Längsteile (14, 15) voneinander entfernt sind, und einer Position, in der die zweiten Enden (24, 37) der Längsteile (14, 15) einander genähert sind, zueinander schwenken können.

3. Befestigungsvorrichtung nach Anspruch 1 oder 2, **dadurch gekennzeichnet, dass** das zweite Längsteil (15) einen ersten, gegenüber der zweiten Fläche (20) des ersten Längsteils (14) befindlichen Abschnitt sowie einen zweiten, gebogenen, das erste Ende (35) des zweiten Längsteils (15) bildenden Abschnitt aufweist.

4. Befestigungsvorrichtung nach Anspruch 3, **dadurch gekennzeichnet, dass** der erste Abschnitt eine erste Bohrung aufweist, die ihn vollkommen durchquert und gegenüber dem ersten Ende der zweiten Fläche des ersten Längsteils (14) mündet.

5. Befestigungsvorrichtung nach Anspruch 2 und 4, **dadurch gekennzeichnet, dass** die zweiten Mittel zum festen Verbinden von einem schraubbaren Element (110) gebildet sind, das geeignet ist, mit einem Innengewinde (112) zusammenzuwirken, welches in die Wand der ersten Bohrung (46) eingebracht ist, wobei das schraubbare Element (110) geeignet ist, sich an dem ersten Ende (114) der zweiten Fläche (20) des ersten Längsteils (14) abzustützen.

6. Befestigungsvorrichtung nach irgendeinem der Ansprüche 1 bis 5, **dadurch gekennzeichnet, dass** die ersten Mittel zum festen Verbinden und Einstellen eine Schraube (28) aufweisen, die ausgebildet ist, um die zweiten Enden (24, 37) des ersten (14) und zweiten (15) Längsteils zu durchqueren, deren Kopf (42) an dem Ende (37) des zweiten Längsteils (15) anliegt und deren Körper in ein Innengewinde (26) des zweiten Endes (24) des ersten Längsteils (14) eingeschraubt ist.

7. Befestigungsvorrichtung nach Anspruch 6, **dadurch gekennzeichnet, dass** das zweite Ende (37) des zweiten Längsteils (15) eine U-förmige Aussparung (116) aufweist, die in entgegengesetzter Richtung zu dem ersten Ende (35) mündet, wobei die Schraube (28) das zweite Ende (24) des ersten Längsteils (14) geneigt durchquert, derart dass der Schraubenkopf (42) von dem ersten Ende (35) entfernt ist, wenn sie sich in einem gelösten Zustand befindet und dass er in einem eingeschraubten Zustand dem ersten Ende (35) genähert ist, wodurch die Schraube (28) in der Lage ist, in die U-förmige Aussparung (116) eingeführt zu werden, wobei der Schraubenkopf (42) in Anlage an dem Umfang der Aussparung (116) ist.

8. Befestigungsvorrichtung nach irgendeinem der Ansprüche 1 bis 7, **dadurch gekennzeichnet, dass** das erste Ende (18) der ersten Fläche (16) eine Kugelkappe bildet, deren Boden eine Bohrung (30) aufweist, die in das Ende der zweiten Fläche (20) des Längsteils (14) mündet, wobei die Kugelkappe ausgebildet ist, um sich an den Schraubenkopf (10) anzulegen.

9. Befestigungsvorrichtung nach irgendeinem der Ansprüche 1 bis 8, **dadurch gekennzeichnet, dass** das erste Ende (35) des zweiten Längsteils (15) einen abgestumpften Kugelkappenabschnitt aufweist, welcher ausgebildet ist, um sich an dem Abschnitt des zweiten halbkugelförmigen Abschnitts (38) des Schraubenkopfes (10) anzulegen.

10. Befestigungsvorrichtung nach irgendeinem der Ansprüche 1 bis 9, **dadurch gekennzeichnet, dass** der Mittelteil (22) der zweiten Fläche (20) eine erste Aufnahme aufweist, in welcher sich der Abschnitt eines ersten Teils der Stange abstützen kann.

11. Befestigungsvorrichtung nach irgendeinem der Ansprüche 1 bis 10, **dadurch gekennzeichnet, dass** der Mittelteil (36) des zweiten Längsteils (15) eine zweite Aufnahme (44) aufweist, die der ersten Aufnahme gegenüberliegt, wobei die zweite Aufnahme (44) ausgebildet ist, um die Stange (12) aufzunehmen.

12. Befestigungsvorrichtung nach irgendeinem der Ansprüche 1 oder 11, **dadurch gekennzeichnet, dass** das erste Längsteil (14) außerdem eine zweite Bohrung (120) aufweist, die zwischen dem Mittelteil (22) und dem zweiten Ende (24) des ersten Längsteils (14) gelegen ist, die in die erste (16) bzw. zweite (20) Fläche mündet und die geeignet ist, eine Schraube (122) aufzunehmen, deren Kopf (124) sich an die zweite Fläche anlegt, um das erste Längsteil (14) in fester Position gegenüber einer Wand zu halten, mit der ersten Fläche (16) an der besagten Wand.

13. Aufbau zur Stabilisierung der Wirbelsäule, **dadurch gekennzeichnet, dass** er folgendes aufweist:
- zwei Pedikelschrauben mit kugelförmigem Kopf (50, 56), die ausgebildet sind, um jeweils in einen Pedikel eines Wirbels (V1) geschraubt zu werden;
- zwei Vorrichtungen (58, 64) nach irgendeinem der Ansprüche 1 bis 11, die an den Schraubenköpfen (50, 56) angebracht sind, wobei die Längsteile zueinander und zur Achse der Wirbelsäule im Wesentlichen parallel sind;
- eine Querstange (66), welche ausgebildet ist, um die zwei Vorrichtungen (58, 64) miteinander zu verbinden, wobei die Stange (66) im Wesentlichen senkrecht zur Achse der Wirbelsäule ist; und
- ein Verbindungssystem (74, 80), welches ausgebildet ist, um die Querstange (66) mit einer zur Achse der Wirbelsäule im Wesentlichen parallelen Längsstange (70,72) zu verbinden.

14. Aufbau zur Stabilisierung der Wirbelsäule, **dadurch gekennzeichnet, dass** er folgendes aufweist:
- zwei Pedikelschrauben mit kugelförmigem Kopf, die ausgebildet sind, um in die Pedikel von zwei übereinander angeordneten Wirbeln geschraubt zu werden;
- zwei Befestigungsvorrichtungen nach irgendeinem der Ansprüche 1 bis 11, die an den Schraubenköpfen angebracht sind, wobei die Längsteile im Wesentlichen parallel zueinander und senkrecht zur Achse der Wirbelsäule sind; und
- eine Längsstange, welche ausgebildet ist, um die Vorrichtungen miteinander zu verbinden, wobei die Längsstange im Wesentlichen parallel zur Achse der Wirbelsäule ist.

15. Aufbau zur Stabilisierung der Wirbelsäule, **dadurch gekennzeichnet, dass** er folgendes aufweist:
- wenigstens zwei Pedikelschrauben (84, 82) mit kugelförmigem Kopf; die ausgebildet sind, um jeweils in einen Pedikel eines Wirbels geschraubt zu werden;
- zwei Vorrichtungen (86, 88) nach irgendeinem der Ansprüche 1 bis 11, die an den Schraubenköpfen (84, 82) angebracht sind, wobei die zweiten Enden (90, 92) der Längsteile zueinander gerichtet sind; und
- ein Querverbindungsteil (94), welches ausgebildet ist, um die beiden Vorrichtungen (86, 88) miteinander zu verbinden, wobei das Verbindungsteil mit wenigstens einem zweiten Ende (90, 92) der Teile der beiden Vorrichtungen (86, 88) fest verbunden ist.

16. Aufbau zur Stabilisierung der Wirbelsäule, **dadurch gekennzeichnet, dass** er folgendes aufweist:
- wenigstens zwei hintere Schrauben mit kugelförmigem Kopf, welche geeignet sind, in zwei übereinander angeordnete Wirbeln jeweils in die hintere Seitenwand des Wirbelkörpers des Wirbels eingeschraubt zu werden; und
- wenigstens zwei Befestigungsvorrichtungen nach Anspruch 12, wobei die ersten Längsteile geeignet sind, in fester Position im Wesentlichen parallel zueinander und zu den Mittelebenen der Wirbel, an der Seitenwand der Wirbel durch vordere, die zweite Bohrung durchquerende Schrauben gehalten zu werden, wobei die ersten Enden der ersten Flächen jeweils an den kugelförmigen Köpfen anliegen und wobei die zwei Befestigungsvorrichtungen durch eine einzige, jeweils von den Mittelteilen der Längsteile eingeklemmte Stange in fester Position zueinander gehalten werden.
